# DEMANDE DE BREVET EUROPEEN

(11) **EP 0 552 109 A1**
(43) Date de publication de la demande: **21.07.1993**
(21) Numéro de dépôt: 93400100.9
(22) Date de dépôt: 18.01.1993
(51) Int. Cl.: A61B 17/064

(54) **Agrafe pour la fixation sur une paroi osseuse**

(30) Priorité: 17.01.1992 FR 9200498
(71) Demandeur: CENDIS MEDICAL SARL, F-75014 Paris (FR)
(72) Inventeur: Orengo, Philippe, F-95300 Cergy Pontoise (FR)
(74) Mandataire: Hasenrader, Hubert

(57) **Abrégé**

Agrafe pour la fixation sur une paroi osseuse comprenant une tête 2 formant un pont entre deux branches verticales 3a,3b se terminant en pointes sur lesquelles sont réalisés des crans de retenue 4, caractérisée en ce que ladite tête 2 comporte une face supérieure 2a qui est réalisée au moins partiellement avec une courbure convexe dont la projection dans un plan horizontal est une ellipse.

## Description

La présente invention concerne une agrafe pour la fixation sur une paroi osseuse.

Les agrafes connues jusqu'à présent sont généralement fxées sur une paroi osseuse au moyen d'un outil porte-agrafe. Cet outil possède à une extrémité un organe d'accrochage pour la tête d'une agrafe et à une autre extrémité une zone de frappe permettant à l'opérateur d'enfoncer l'agrafe en force dans la zone osseuse.

L'agrafe pénètre dans l'os par deux branches verticales se terminant en pointes, et pourvues de crans de retenue et de blocage.

Cependant la frappe sur l'outil provoque le plus souvent une déformation de la tête de l'agrafe qui possède des arêtes vives.

De plus, la liaison entre l'outil et l'agrafe n'est pas toujours satisfaisante dans la mesure où les chocs dus à la frappe ont tendance à la perturber, à la fausser et à rendre difficile la séparation ultérieure de l'agrafe.

La présente invention a pour but de remédier à ces inconvénients ou du moins de les atténuer.

Ce but est atteint au moyen d'une agrafe pour la fixation sur une paroi osseuse comprenant une tête formant un pont entre deux branches verticales se terminant en pointes sur lesquelles sont réalisés des crans de retenue, caractérisé en ce que ladite tête comporte une face supérieure à courbure convexe dont la projection dans un plan horizontal est une ellipse.

Selon une caractéristique avantageuse, la tête comporte dans sa partie centrale un orifice traversant taraudé dans lequel vient se visser l'organe fileté d'accrochage de l'outil porte-agrafe.

Selon une autre caractéristique, la face inférieure de la tête entre les deux branches est pourvue de doigts verticaux de calage.

Selon encore une autre caractéristique, les crans de retenue sont réalisés sur les faces latérales externes des branches verticales et la face inférieure de l'agrafe s'étend dans un plan sensiblement horizontal.

L'agrafe de l'invention n'est pratiquement pas déformée lors de sa fixation en force et la séparation entre l'outil et l'agrafe s'effectue aisément.

De plus, le profil elliptique et convexe de la tête de l'agrafe la rend beaucoup moins agressive et évite ainsi tout problème de blessure et d'infection.

L'invention sera mieux comprise à la lecture de la description qui va suivre accompagnée des dessins sur lesquels ;
- la figure 1 représente une vue en perspective de l'agrafe de l'invention avec une vue partielle de son outil de support.

La figure 2 représente une vue de dessus de l'agrafe de la figure 1.

Les figures 3a et 3b représentent des vues en perspective d'autres modes de réalisation de l'invention.

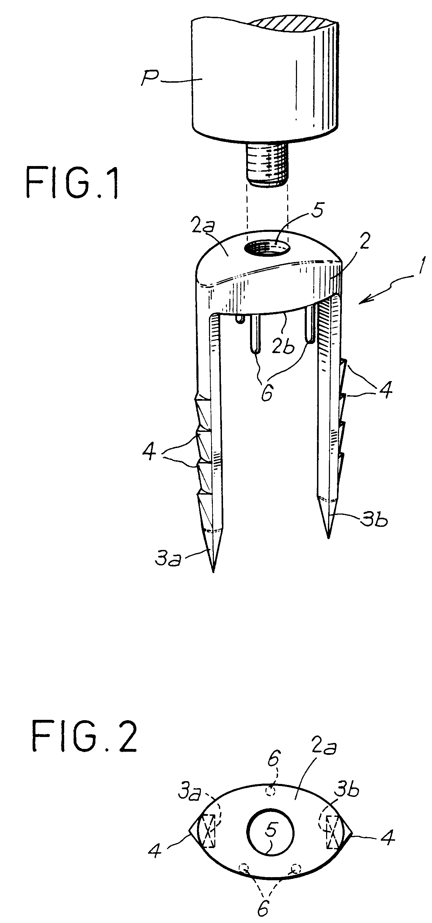

L'agrafe représentée sur la figure 1 présente une forme générale en U et comprend une tête 2 formant un pont entre deux branches 3a,3b verticales qui se terminent en pointes et dont l'écartement est compris entre 3 et 10 mm.

Les branches 3a,3b comportent des crans de retenue 4 réalisés sur leur face latérale externe. Les branches 3a,3b ont une section transversale sensiblement parallélépipédique et les crans 4 sont réalisés sous forme de dents superposées constitués d'un pan incliné surmonté d'une face horizontale perpendiculaire à la branche 3a,3b.

La tête 2 comporte une face supérieure 2a de forme bombée avec une courbure convexe dont la projection dans un plan horizontal est une ellipse.

Cette courbure permet d'obtenir une très faible surface de contact entre l'extrémité d'accrochage de l'outil de support P et le sommet de la tête 2 de l'agrafe.

Ainsi lors de la frappe sur l'outil en vue de la fixation de l'agrafe, les déformations de la tête sont elles extrêmement limitées.

Dans la partie centrale, située au sommet de la tête 2 se trouve un orifice 5 traversant taraudé dans lequel vient se visser l'organe fileté d'accrochage de l'outil porte-agrafe P.

La face inférieure 2b de la tête 2 est sensiblement horizontale et est pourvue à sa périphérie de doigts verticaux de calage 6 destinés à s'enfoncer dans la couche superficielle de la paroi osseuse.

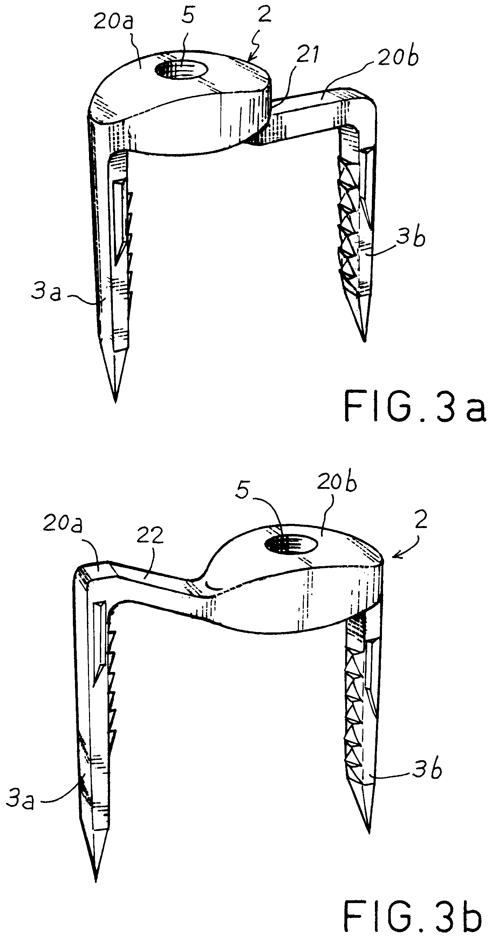

L'agrafe représentée sur la figure 3a comporte des branches verticales 3a,3b de longueurs différentes ; la branche 3a étant plus longue que la branche 3b.

Par suite, la tête 2 est constituée de parties horizontales 20a,20b reliées par un tronçon vertical 21, dont la hauteur correspond sensiblement à la différence de longueur entre les branches.

Dans ce mode de réalisation, la partie horizontale la plus haute 20a est réalisée entre une courbure convexe dont la projection dans un plan horizontal est une ellipse.

La partie 20a comporte également comme dans les modes de réalisation des figures précédentes, un orifice traversant taraudé 5 dans lequel vient se visser l'organe fileté d'accrochage de l'outil P.

Dans le mode de réalisation de la figure 3b, les parties horizontales 20a, 20b sont reliées par un tronçon incliné 22. La partie horizontale 20b la plus basse est dans ce cas celle qui possède la plus grande surface et c'est elle qui est réalisée avec une face supérieure de courbure convexe dont la projection dans un plan horizontal est une ellipse.

La partie 20b comporte aussi un orifice traversant taraudé 5 adapté à l'outil P.

Dans les modes de réalisation des figures 3a et 3b, les crans de retenue 4 sont de préférence réalisés sur les faces latérales internes des branches 3a,3b.

## Revendications

1. Agrafe pour la fixation sur une paroi osseuse comprenant une tête 2 formant un pont entre deux branches verticales 3a,3b se terminant en pointes sur lesquelles sont réalisés des crans de retenue 4, caractérisée en ce que ladite tête 2 comporte une face supérieure 2a qui est réalisée au moins partiellement avec une courbure convexe dont la projection dans un plan horizontal est une ellipse.

2. Agrafe selon la revendication 1, caractérisée en ce que ladite tête 2 comporte dans sa partie centrale un orifice traversant taraudé 5 dans lequel vient se visser l'organe fileté d'accrochage de l'outil porte-agrafe P.

3. Agrafe selon l'une des revendications 1 ou 2, caractérisée en ce que la face inférieure 2b de la tête 2 entre les deux branches 3a,3b est pourvue de doigts verticaux 6 de calage.

4. Agrafe selon l'une des revendications précédentes, caractérisée en ce que lesdits crans de retenue 4 sont réalisés sur les faces latérales externes des branches verticales 3a,3b.

5. Agrafe selon l'une des revendications précédentes, caractérisé en ce que la face inférieure 2b de la tête 2 s'étend dans un plan sensiblement horizontal.

6. Agrafe selon la revendication 1 ou 2, caractérisée en ce que les deux branches verticales 3a,3b sont de longueurs différentes et la tête 2 formant pont comporte des parties horizontales 20a,20b et des tronçons verticaux 21 et/ou inclinés 22 ; lesdites parties horizontales 20a,20b étant réalisées avec une courbure convexe dont la projection dans un plan horizontal est une ellipse.

7. Agrafe selon la revendication 6, caractérisé en ce que les crans de retenue 4 sont réalisés sur les faces latérales internes des branches 3a,3b.
